**Europäisches Patentamt**

**European Patent Office**

**Office européen des brevets**

(19)

(11) Veröffentlichungsnummer: **0 176 719**
**B1**

(12)

# EUROPÄISCHE PATENTSCHRIFT

(45) Veröffentlichungstag der Patentschrift:
**14.09.88**

(51) Int. Cl.⁴: **C 12 M 1/28**, B 65 D 51/00

(21) Anmeldenummer: **85110251.7**

(22) Anmeldetag: **16.08.85**

(54) **Blutkulturflasche mit integrierter Subkultur.**

(30) Priorität: **25.08.84 DE 8425171 U**

(43) Veröffentlichungstag der Anmeldung:
**09.04.86 Patentblatt 86/15**

(45) Bekanntmachung des Hinweises auf die Patenterteilung:
**14.09.88 Patentblatt 88/37**

(84) Benannte Vertragsstaaten:
**AT BE CH DE FR GB IT LI LU NL SE**

(56) Entgegenhaltungen:
FR - A - 2 292 455
FR - A - 2 381 102
FR - A - 2 381 103
US - A - 2 364 126
US - A - 2 992 974
US - A - 3 499 568

**Die Akte enthält technische Angaben, die nach dem Eingang der Anmeldung eingereicht wurden und die nicht in dieser Patentschrift enthalten sind.**

(73) Patentinhaber: **Biotest Aktiengesellschaft, Flughafenstrasse 4, D-6000 Frankfurt 71 (DE)**

(72) Erfinder: **Hempel, Hans Dieter, Dipl.-Ing., Froschgraben 19, D-8752 Mainaschaff (DE)**
Erfinder: **Horn, Jürgen, Dipl.-Chem., Kurt-Schumacherring 83, D-6073 Egelsbach (DE)**
Erfinder: **Rothermel, Wilfried, Dipl.-Chem., Auestrasse 49, D-6057 Dietzenbach (DE)**
Erfinder: **Sonneborn, Hans Helmut, Dipl.-Biol., Im Birkeneck 70, D-6056 Heusenstamm (DE)**
Erfinder: **Becker, Michael, Buchrainweg 59, D-6050 Offenbach (DE)**
Erfinder: **Müller, Ullrich, Dipl.-Biol., Waldgartenstrasse 37, D-6090 Rüsselsheim (DE)**

(74) Vertreter: **Wolff, Hans Joachim, Dr.jur. Dipl.-Chem. et al, Beil, Wolff & Beil Rechtsanwälte Postfach 80 01 40 Adelonstrasse 58, D-6230 Frankfurt am Main 80 (DE)**

## Beschreibung

Die Erfindung betrifft eine Blutkulturflasche mit integrierter Subkultur nach dem Oberbegriff des Anspruchs 1.

Zum Nachweis von Mikroorganismen in Körperflüssigkeiten, insbesondere von Bakterien im Blut, ist es allgemein üblich, flüssiges Nährmedium mit Patientenblut zu beimpfen, um evtl. vorliegende Erreger anzureichern und anschliessend das Wachstum auf einem festen Nährboden zu ermöglichen.

Seit langem gibt es für diesen Zweck sogenannte zweiphasige Blutkulturflaschen, bei denen beide Nährmedien im gleichen Behälter in einem geschlossenen System kombiniert sind, um die sonst übliche arbeitsintensive Subkultivierung und das nicht auszuschliessende Kontaminationsrisiko zu vermeiden.

Bei diesen Kulturflaschen handelt es sich um Dreikant-, Vierkant- oder Sechskant-Glasflaschen, die etwa bis zur Hälfte oder einem Drittel mit flüssigem Nährmedium gefüllt sind und bei denen eine Seitenwand mit einer festen Nährbodenphase beschichtet ist. Alle Flaschen sind mit irgendeiner Art luftdichtem Verschluss versehen, der von einer Injektionsnadel oder ähnlichem Gerät durchdrungen werden kann und beim Beimpfen nicht geöffnet werden muss. Derartige Flaschen werden in Castaneda-Ruiz, M. (1947) Practical Method for routine blood cultures in brucellosis Proc. Soc. Exp. Biol. Med. 64: 114–115 und in verschiedenen Firmendruckschriften als Castaneda-Flasche, Hemoline-Trypcase zweiphasig oder Vacuneda-Flasche beschrieben.

Bei den oben beschriebenen Systemen erstreckt sich die feste Nährbodenphase jeweils über die gesamte Länge einer Seitenwand bis hinein in die flüssige Phase und steht somit bei aufrechter Stellung der Flasche ständig mit der flüssigen Phase in Berührung. Durch diesen ständigen Phasenkontakt erfolgt ein Austausch der Inhaltsstoffe, wodurch die flüssige Nährlösung trübe wird und die Farbe und/oder Konsistenz der festen Nähragarschicht sich ändert. Besonders kritisch ist dieser Vorgang bei Verwendung von Kochblutagar, der durch die flüssige Phase besonders stark ausgelaugt wird. Obgleich Kochblutagar für Blut-Subkulturen ein besonders wertvoller Nähragar ist, insbesondere zur Anzüchtung anspruchsvoller Keime, ist er in den oben beschriebenen geschlossenen Zweiphasensystemen aus den vorstehend genannten Gründen nicht verwendbar.

Dieser Mangel wird zwar gemäss DE-GM 83 09 876 durch eine zweiphasige Blutkulturflasche dadurch beseitigt, dass die Flasche eine als erkerartige Ausbuchtung ausgebildete Nährbodenfuge im oberen Teil einer Seitenwand der Flasche aufweist, so dass die darin befindliche feste Nährbodenphase über dem Spiegel des flüssigen Nährmediums liegt und somit ein ungewollter Phasenkontakt in aufrechter Stellung vermieden wird, jedoch ist die Herstellung einer solchen Flasche und auch anderer der oben beschriebenen Kulturflaschen, soweit sie mit Einkerbungen und inneren Rippen zum Festhalten des Nährbodens ausgebildet sind, relativ aufwendig und kostspielig.

Ausserdem besitzen diese Art von Kulturflaschen alle den gemeinsamen Nachteil, dass die feste Nährbodenphase nach dem Bebrüten der Flasche nicht herausgenommen werden kann.

In der DE-PS 28 06 902 wird ein System beschrieben, das aus zwei getrennten Behältern besteht, wobei sich in einem Behälter das flüssige Nährmedium und im anderen die feste Nährphase befindet. Beide Behälter sind vor der Ingebrauchsnahme voneinander getrennt und jeweils verschlossen. Bei Ingebrauchsnahme müssen beide Behälter geöffnet werden und werden nach Beimpfung des flüssigen Nährmediums mit Hilfe eines Dichtungsringes, vorzugsweise aus Polyethylen, miteinander verbunden. Das Behältnis mit dem festen Nährmedium befindet sich dabei relativ hoch über dem anderen Behälter. Als Träger für das feste Nährmedium dient eine Art Objektträger.

Mit diesem System wird zwar sowohl der Nachteil der ständigen Berührung zwischen fester und flüssiger Phase und der aufwendigen Herstellung beseitigt, jedoch besitzt dieses System einige andere Nachteile. Durch die Notwendigkeit des Öffnens beider Behälter nach der Beimpfung besteht die grosse Gefahr einer Sekundärkontamination.

Durch das erforderliche Öffnen des Behälters mit dem flüssigen Medium gelangt Luft in den Behälter und verhindert den Nachweis obligat anaerober Erreger. Auch kapnophile Erreger, die $CO_2$-abhängig sind, können nicht nachgewiesen werden, da der Träger mit dem festen Nährboden sich nur im oberen Teil befindet, während das schwerere $CO_2$ nur weit unten vorhanden ist. Es findet in diesem System deshalb ein sehr schlechtes Wachstum von Anaerobiern und $CO_2$-abhängigen Erregern statt.

In der DE-OS 19 59 902 wird eine geschlossene zweiphasige Blutkulturflasche beschrieben, in die eine Trägerschale zur Aufnahme von festem Nährboden eingeschoben und das ganze mit Hilfe eines Gummistopfens und einer Schraubkappe verschlossen wird.

Hierbei handelt es sich somit um ein geschlossenes System, das im evakuierten Zustand beimpft und sowohl belüftet als auch unbelüftet bebrütet werden kann und aus dem nach dem Bebrüten die feste Nährbodenphase herausgenommen werden kann. Die Trägerschale besteht aus Giessharz, Glas oder einem anderen keramischen Material und weist einen hohlzylindrischen Endabschnitt auf, der mit Reibschluss in der Öffnung des Flaschenhalses angeordnet ist. Der Hohlraum des Zylinders wird mit einem elastischen Stopfen ausgefüllt, dessen überstehender Rand auf dem oberen Rand des zylindrischen Endabschnitts aufliegt. Das ganze kann mit einer Schraubkappe abgedeckt werden. Diese Kulturflasche besitzt jedoch den Nachteil, dass sich mittels Reibschluss zwischen zylindrischem Endabschnitt der Trägerschale und dem Flaschenhals, beides nichtelastische Materialien, keine 100%ige Dichtigkeit der Flasche erzielen lässt. Auch der darüberliegende überstehende Rand des elastischen Stopfens reicht nicht aus, um die Flasche völlig abzudichten, so dass in eine evakuierte Flasche oder eine Flasche, die ein bestimmtes Gas enthält, im Laufe der Zeit Luft eindringt bzw. das Gas entweicht.

Da die Trägerschale sich über die ganze Länge der Flasche erstreckt, besitzt diese Kulturflasche ausserdem den anfänglich für bekannte zweiphasige Blutkulturflaschen beschriebenen Nachteil, dass die feste Nährbodenphase bei aufrechter Stellung ständig mit der flüssigen Phase in Berührung steht.

Ein weiterer Nachteil besteht darin, dass die Trägerschale nur einseitig zu beschichten ist und in Fällen, in denen diese eine Seite nicht durch Trennwände unterteilt ist, keine unterschied-lichen Nährbodenzusammensetzungen aufge-bracht werden können. Für ein selektives Keimwachstum ist dies aber notwendig. Es ist dort zwar eine Ausführungsform vorgesehen, bei der die Trägerschale durch Stege in verschiedene Abteile unterteilt ist. Die Flächen der Abteile sind dabei jedoch so klein, dass diese Ausführungsform sich für die praktische Anwendung als ungeeignet erwies.

Schliesslich besteht ein weiterer Nachteil darin, dass der aus Schale und hohlzylindrischem End-abschnitt bestehende Träger vom fertigungstech-nischen Standpunkt aus äusserst kompliziert ist und somit seine Herstellung relativ aufwendig ist.

Der Erfindung liegt die Aufgabe zugrunde, eine Blutkulturflasche mit integrierter Subkultur als geschlossenes System zu schaffen, d.h. die nicht die Nachteile eines offenen Systems aufweist, wie u.a. Gefahr einer Sekundärkontamination und Veränderung der atmosphärischen Konsistenz beim notwendigen Öffnen der Flasche, und darüberhinaus die Nachteile der bisher bekannten geschlossenen Systeme vermeidet, d.h. eine Flasche, in der feste und flüssige Phase sicher voneinander getrennt sind, der feste Nährboden, falls gewünscht, der Flasche zu entnehmen ist und die evakuiert werden kann und darüberhinaus einen Nährbodenträger mit mindestens zwei ausreichend grossen zu beschichtenden Flächen aufweist, dessen Nährboden auch bei aufrechter Stellung nicht mit der Nährlösung in Berührung kommt, und der fertigungstechnisch einfach und preisgünstig ist, und die vollständig dicht zu verschliessen ist und im evakuierten Zustand beimpft und bebrütet werden kann.

Diese Aufgabe wird bei einer gattungsgemässen Blutkulturflasche durch die kennzeichnenden Merkmale des Anspruchs 1 gelöst.

Ein wesentliches Element der erfindungsge-mässen Blutkulturflasche ist das Verschlusssystem mit integriertem Nährbodenträger, d.h. das Zusammenspiel zwischen Schraubkappenunterteil, Schraubkappenoberteil, elastischem Stopfen mit daran befestigtem Nährbodenträger und Flaschenhals der Flasche. Das als Hohlzylinder aus-gebildete Schraubkappenunterteil besitzt in sei-nem unteren Abschnitt ein Innengewinde zum Aufschrauben auf das Aussengewinde des Flaschenhalses. Der Stopfen besitzt einen oberen überstehenden elastischen Rand, einen sogenannten Hut. Der Durchmesser des Hutes entspricht dem Innendurchmesser des gewindefreien Schraubkappenunterteils, so dass die untere Fläche seines oberen überstehenden elastischen Randes oder Hutes auf einem Absatz oberhalb der oberen Kante des Innengewindes aufliegt. Der Durchmesser des unteren Stopfenteils ist um soviel dicker als der Innendurchmesser des Flaschenhalses, wie erforderlich ist, um ihn dichtsitzend in den Flaschenhals einpressen zu können. Der elastische Stopfen kann beispielsweise aus Naturkautschuk, synthetischem Kautschuk oder einem elastischen Kunststoff bestehen.

Das Schraubkappenoberteil lässt sich fest auf das Schraubkappenunterteil aufbringen, vorzugsweise aufschrauben, und ist so gestaltet, dass es im aufgebrachten Zustand Druck auf den Stopfen ausübt. Eine Schraubverbindung kann zweckmässigerweise entweder mit Hilfe eines Aussengewindes am Schraubkappenoberteil auf ein entsprechendes Innengewinde am oberen Teil des Schraubkappenunterteils erfolgen, oder das Schraubkappenoberteil kann das Schraubkappenunterteil überlappen und mit Hilfe eines Innengewindes an einem Aussengewinde des Schraubkappenunterteils verschraubt werden.

Wesentlich ist dabei, dass im aufgeschraubten Zustand der Absatz oberhalb der oberen Kante des Innengewindes des Schraubkappenunterteils unter der Oberkante des Flaschenhalses zu liegen kommt. Dadurch liegt nun zwangsläufig der elastische Stopfen auf der Oberkante des Flaschenhalses auf. Durch den Druck des Schraubkappenoberteils auf den Stopfen ist letzterer durch den Spielraum zwischen Oberkante des Flaschenhalses und dem besagten Absatz eindrückbar und bildet dadurch einen vollständig luft- und gasdichten Verschluss.

Ein Abstand von 0,5 mm zwischen dem besagten Absatz und der Oberkante des Flaschenhalses genügt bereits, um diese Wirkung zu erzielen.

In einer bevorzugten Ausführungsform weist der Flaschenhals unterhalb des Aussengewindes einen Absatz auf, der als Anschlag für das Schraubkappenunterteil dient. Dadurch lässt sich der Abstand zwischen Flaschenoberkante und oberer Gewindekante des Innengewindes des Schraubkappenunterteils besser fixieren.

Ein weiterer Vorteil der erfindungsgemässen Anordnung besteht darin, dass beim Herausdrehen der Schraubkappe der auf dem Absatz oberhalb der oberen Kante des Innengewindes aufliegende elastische Stopfen mit angehoben wird, d.h. zusammen mit dem Nährbodenträger leicht und ohne Berührung entfernt werden kann.

Ausser als Druckelement auf den elastischen Stopfen dient das Schraubkappenoberteil als Abdeckung der Stichfläche des Stopfens, wodurch keine Verunreinigung derselben erfolgen kann.

Als Nährbodenträger eignen sich flache Platten wie sie unter dem Begriff Dip-slide oder Keimindikatoren bekannt sind. Die Länge des Trägers darf nur derart sein, dass er sich nur über einen Teil der Länge der Flasche erstreckt. In jedem Fall muss er oberhalb des Spiegels der in der Flasche befindlichen Nährflüssigkeit enden. Zwischen Flüssigkeitsspiegel und Ende des Trägers sollte soviel Spielraum sein, dass beim Transportschütteln kein Kontakt zwischen fester und flüssiger Phase erfolgt. Ein solcher Träger ist beidseitig mit Nährboden zu beschichten, wodurch mindestens zwei selektive Phasen geschaffen werden. Falls man mehr als zwei Phasen wünscht, kann der Träger in Längs- oder Querrichtung auf einer oder beiden Seiten durch einen Trennungssteg unterteilt sein.

Als besonders zweckmässig erwies sich ein Träger mit einer glatten Oberfläche, um einer Bläschenbildung beim Evakuieren keine Angriffspunkte zu geben, und mit den Nährboden überschneidenden Halteflächen, um ein Abrutschen des Nährbodens beim Benetzen zu verhindern. Die Halteflächen können an allen vier Seiten des Trägers angebracht sein, können sich jedoch nur an einer Schmalseite und den zwei Längsseiten des Trägers oder nur an den zwei Längsseiten befinden. Sie können durchgehend oder unterbrochen sein.

Als feste Nährböden lassen sich alle für diesen Zweck üblichen, beispielsweise Agar, Nährböden verwenden. Als besonders geeignet erwiesen sich Agarnährböden, denen Gellan Gummi, ein Polysaccharid, zugesetzt wurde. Ganz besonders gut eignete sich dabei Gellan Gummi mit niedrigem Acetylgehalt und insbesondere letzteres mit hoher Klarheit, wie es unter der Handelsbezeichnung GELRITE® vertrieben wird (vergl. J.K. Baird et.al., «Industrial applications of some new microbial polysaccharides», Biotechnology November 1983, S. 778–783).

Nährböden, die beispielsweise 10 g Agar + 4 g GELRITE/l enthielten, erwiesen sich auf den erfindungsgemäss eingesetzten Trägern als sehr gut haftend, während bei Verwendung von reinem Agar eine Menge von 19 g/l erforderlich ist, um Haftung zu erzielen.

Es erfolgt ausserdem keine Spaltbildung entlang der Hinterschneidung der Halteflächen. Ausserdem erwies sich diese Art von Nährböden als formstabiler auf dem Träger innerhalb der Blutkulturflasche, insbesondere beim Evakuieren der Flasche. Schliesslich beobachtete man besseres Wachstum (grössere Kolonien) bei einigen Keimen gegenüber dem Wachstum auf reinem Agar.

Sollte das flüssige Nährmedium wider Erwarten doch mit dem Träger in Kontakt kommen, so können bei Nährböden, welche niedermolekulare Substanzen enthalten diese eluiert werden. Der Nährboden, insbesondere der vorstehend beschriebene, bleibt zwar trotzdem haften, hat dann aber nicht mehr seine typischen Eigenschaften, z.B. wachsen bei einem McConkey die coliformen Keime nicht mehr typisch, da dann Gallensalze und Kristallviolett eluiert werden. Zur Verhinderung dieses Ausblutens der niedermolekularen Substanzen und Sicherstellung eines typischen Wachstums erwies sich die Verwendung einer Nährbodenkombination als besonders geeignet, bei der eine Schicht McConkey mit Zusatz von Gellan Gummi, vorzugsweise GELRITE®, und der etwa 2–8fachen Konzentration der üblichen Menge an niedermolekularen Substanzen wie z.B. Gallensalzen und Kristallviolett auf den Träger aufgebracht wird und man darüber eine Deckschicht aus normalem Agar bzw. McConkey in Normalzusammensetzung aufbringt.

Zweckmässigerweise weist der Träger am unteren Ende eine Tropfnase auf, damit überschüssige Nährlösung nach dem Benetzen besser abtropfen kann.

Als Befestigungsmittel des Trägers am elastischen Stopfen eignen sich besonders ein oder vorzugsweise zwei stangenartige Ansätze an einer Schmalseite des Trägers, die in entsprechende Löcher im Stopfen eingesteckt werden. Um einen besonders festen Halt zu gewährleisten, sollten die Stangen Verdickungen und die Löcher im Stopfen Hinterschneidungen aufweisen. Das Stangenprofil kann jede beliebige geeignete Form aufweisen, wie beispielsweise kreuzförmig, sternförmig, rund oder dergleichen.

Der Träger kann aus jedem den Nährmedien gegenüber inertem, formbaren Material sein. Zweckmässigerweise ist er aus einem gegenüber der festen und der flüssigen Nährphase inerten Kunststoff.

Die Flasche als solche kann jede Art von üblicher Blutkulturflasche sein. Sie kann rund oder eckig sein, kann aus Glas oder einem transparenten Kunststoff sein. Vorzugsweise ist sie jedoch aus Glas, da Glas eine geringere Gaspermeabilität aufweist als Kunststoff. Sie sollte so gross sein, dass die übliche Menge an Nährlösung, die für Blutkulturen verwendet wird, d.h. ca. 40–100 ml, möglichst nicht mehr als die Hälfte der Höhe der Flasche einnimmt, damit genügend Freiraum für den Nährbodenträger bleibt. Der Flaschenhals muss rund und so weit sein, dass der Nährbodenträger eingeführt werden kann. Er weist ein Aussengewinde auf, zweckmässigerweise ein normales DIN-Aussengewinde.

Aufgrund der Tatsache, dass sich die erfindungsgemässe Flasche im evakuierten Zustand beimpfen und bebrüten lässt, ist sie besonders für anaerobe Erreger geeignet. Wahlweise kann ein Rest Luft- oder Gasgehalt im System verbleiben.

Nachstehende Zeichnungen dienen der weiteren Erläuterung der Erfindung.

Es zeigen

Fig. 1 einen Aufriss der Flasche mit integriertem Träger;

Fig. 2 einen Aufriss des Verschlussystems im Zusammenspiel mit dem Flaschenhals;

Fig. 3 einen Aufriss eines Trägers;

Fig. 4 einen Schnitt durch den Träger;

Fig. 5 einen Aufriss des elastischen Stopfens mit eingesteckten stangenartigen Ansätzen des Trägers;

Fig. 6 eine Draufsicht auf den elastischen Stopfen.

7     **0 176 719**     8

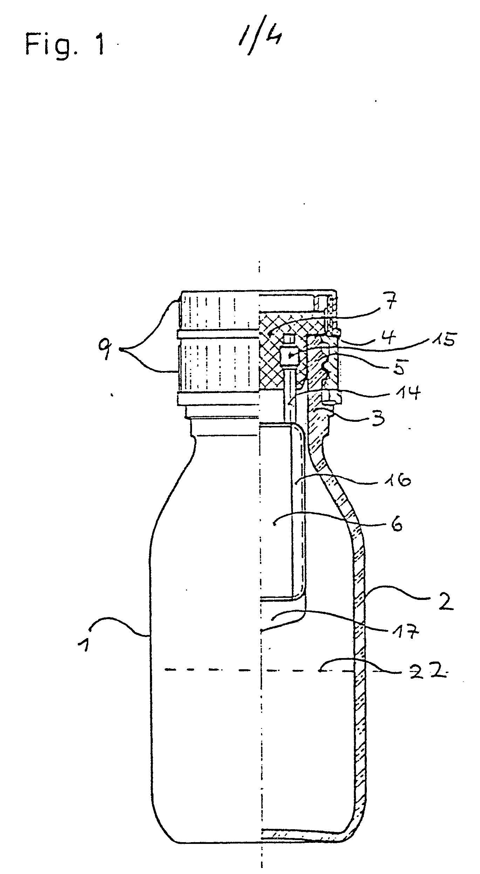

Fig. 1 zeigt die Blutkulturflasche (1) mit Flasche (2), Flaschenhals (3), Oberkante (4) des Flaschenhalses und Aussengewinde (5). Träger (6) mit Halteflächen (16), Tropfnase (17) und Verdickungen (15) aufweisenden Ansätzen (14) ist in den elastischen Stopfen (7) eingesteckt.

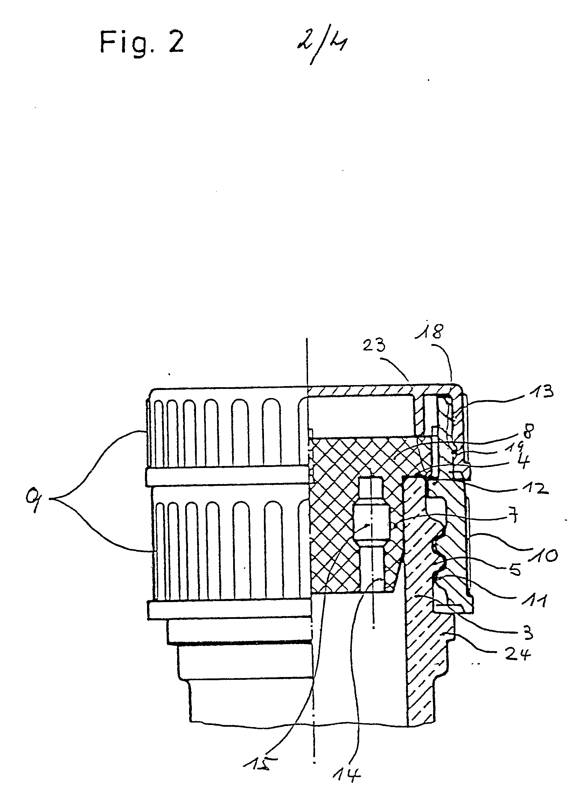

Flüssigkeitsspiegel (22) befindet sich im Abstand unter dem Träger (6). Die Konstruktion des Flaschenhalses (3) und der Schraubkappe (9) wird in Fig. 2 in vergrössertem Massstab gezeigt und dort näher erläutert.

In Fig. 2 ist bei der Schraubkappe (9) das Schraubkappenunterteil (10) mit Hilfe seines Innengewindes (11) auf das Aussengewinde (5) des Flaschenhalses (3) aufgeschraubt. Der Absatz (12) oberhalb der oberen Kante des Innengewindes (11) liegt dabei unter der Oberkante (4) des Flaschenhalses (3). Die Unterfläche des überstehenden elastischen Randes (8) des elastischen Stopfens (7) liegt dabei auf der Oberkante (4) des Flaschenhalses (3). Das Schraubkappenoberteil (13) drückt nun im aufgeschraubten Zustand den überstehenden elastischen Rand (8) in den Spalt (23) zwischen Absatz (12) und Flaschenrand (4).

Dadurch wird ein hermetischer Verschluss erzielt. Die Verbindung zwischen den beiden Schraubkappenteilen erfolgt durch Verschrauben mit Hilfe eines Innengewindes (19) am Schraubkappenoberteil (13) mit einem etwas zurückgesetzten Aussengewinde (18) am Schraubkappenunterteil (10).

Unterhalb des Aussengewindes (5) befindet sich ein Absatz (24), der als Anschlag für das aufzuschraubende Schraubkappenunterteil (10) dient. Ansatz (14) mit Verdickungen (15) des Trägers (6) sind in den elastischen Stopfen (7) eingesteckt.

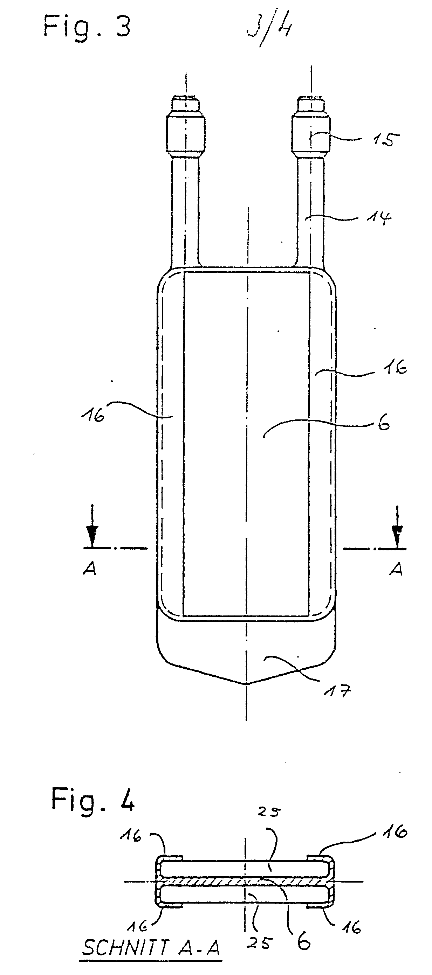

Fig. 3 zeigt den Träger (6) mit Halteflächen (16), stangenartigen Ansätzen (14) mit Verdickungen (15) zum Einstecken in den elastischen Stopfen und Tropfnase (17).

Fig. 4 zeigt einen Schnitt durch den Träger (6) mit Halteflächen (16) und beidseitig aufgebrachtem festen Nährboden (25).

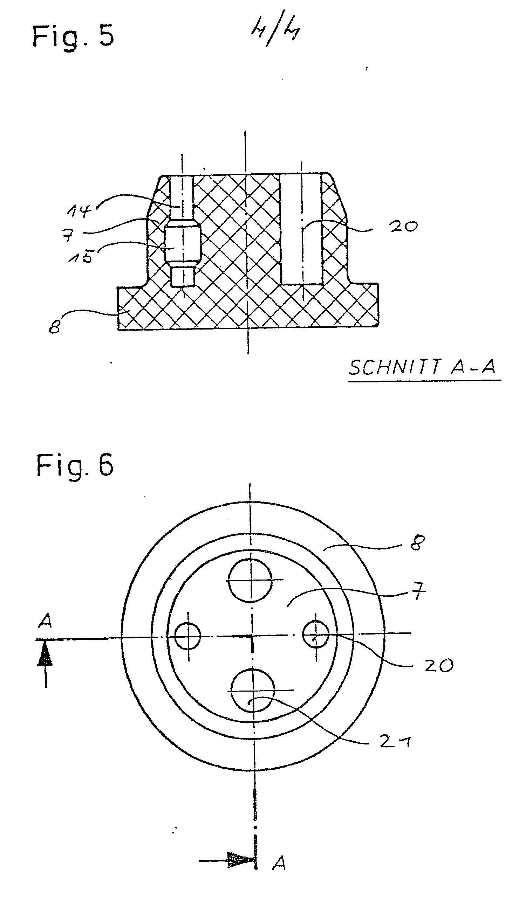

In Fig. 5 sind die stangenartigen Ansätze (14) mit den Verdickungen (15) im eingesteckten Zustand in den Stopfen (7) zu sehen. Ferner sieht man den überstehenden elastischen Rand (8) des Stopfens und Bohrungen (20) zum Einstecken der Ansätze.

In der Draufsicht der Fig. 6 sieht man den elastischen Stopfen (7) mit überstehendem elastischen Rand (8), Bohrungen (20) zum Einstecken des Trägers und Einstichmarkierungen (21) für die Injektionsnadel.

**Patentansprüche**

1. Blutkulturflasche mit integrierter Subkultur, enthaltend eine mit flüssiger Nährlösung füllbare Flasche mit einem Flaschenhals, der ein Aussengewinde aufweist, einen in die Flasche einschiebbaren, mit festem Nährboden zu beschichtenden Träger, einen elastischen Stopfen mit überstehendem Rand auf seiner einen Seite und eine Schraubkappe dadurch gekennzeichnet, dass die Schraubkappe (9) aus einem oben und unten offenen Schraubkappenunterteil (10) mit einem Innengewinde (11) und einem nach unten offenen Schraubkappenoberteil (13) besteht, das Innengewinde (11) so dimensioniert ist, dass es sich auf das Aussengewinde (5) des Flaschenhalses (3) aufschrauben lässt, derart, dass die obere Gewindekante (12) im aufgeschraubten Zustand unterhalb der Oberkante (4) des Flaschenhalses (3) liegt, der elastische Stopfen (7) im Inneren des Schraubkappenunterteils (10) so angeordnet ist, dass die untere Fläche seines überstehenden elastischen Randes (8) im nicht aufgeschraubten Zustand auf dem Absatz (12) oberhalb des Gewindes zu liegen kommt und sein unterer Abschnitt so dimensioniert ist, dass er festsitzend in den Flaschenhals (3) einzuführen ist, der Träger (6) auf gegenüberliegenden Seiten zwei mit festem Nährboden zu beschichtende Flächen aufweist, am unteren Ende des elastischen Stopfens (7) befestigt ist und im Gebrauchszustand sich nur über einen Teil der Länge der Flasche (2) erstreckt und das Schraubkappenoberteil (13) derart ausgebildet ist, dass es im aufgebrachten Zustand den überstehenden elastischen Rand (8) des elastischen Stopfens (7) fest auf die Oberkante (4) des Flaschenhalses (3) drückt.

2. Blutkulturflasche nach Anspruch 1, dadurch gekennzeichnet, dass der Flaschenhals (3) unterhalb des Aussengewindes (5) einen Absatz (24) als Anschlag für das Schraubkappenunterteil (10) aufweist.

3. Blutkulturflasche nach einem der Ansprüche 1 oder 2, dadurch gekennzeichnet, dass Träger (6) an seinem oberen Rand ein oder mehrere, vorzugsweise zwei, stangenartige Ansätze (14) mit Verdickungen (15) und der elastische Stopfen (7) in seiner Unterfläche entsprechende Bohrungen (20) aufweist, in die diese Ansätze (14) zwecks Befestigung eingesteckt werden.

4. Blutkulturflasche nach einem der vorstehenden Ansprüche, dadurch gekennzeichnet, dass die zu beschichtenden Flächen des Trägers (6) glatt sind.

5. Blutkulturflasche nach einem der vorstehenden Ansprüche, dadurch gekennzeichnet, dass die zu beschichtenden Flächen des Trägers mindestens an den beiden Längsseiten Halteflächen (16) für den Nährboden aufweisen, die letzteren überschneiden.

6. Blutkulturflasche nach einem der vorstehenden Ansprüche, dadurch gekennzeichnet, dass der Träger (6) eine Tropfnase (17) aufweist.

7. Blutkulturflasche nach einem der vorstehenden Ansprüche, dadurch gekennzeichnet, dass der feste Nährboden (25) auf dem Träger (6) Gellan Gummi enthält.

8. Blutkulturflasche nach Anspruch 7, dadurch gekennzeichnet, dass der feste Nährboden (25) eine erste, auf den Träger (6) aufgebrachte Schicht aus McConkey mit einem Zusatz an Gellan Gummi und der 2–8fachen Konzentration der normalen Menge an niedermolekularen Substanzen und eine Deckschicht aus normalem Agar

5

und/oder McConkey in Normalzusammensetzung aufweist.

**Revendications**

1. Flacon pour hémo-culture à sub-culture intégrée, comportant un flacon remplissable avec un bouillon de culture liquide, ayant un col qui présente un filetage externe, un support à garnir avec un milieu de culture solide pouvant être glissé dans le flacon, un bouchon élastique avec une collerette débordant sur l'une de ses faces et une capsule vissée, caractérisé en ce que la capsule vissée (9) est constituée par une partie inférieure (10) ouverte en haut et en bas comportant un filetage interne (11) et par une partie supérieure (13) ouverte vers le bas, en ce que le filetage interne (11) est dimensionné de telle manière qu'il soit possible de le visser sur le filetage externe (5) du col (3) du flacon de telle sorte que le bord supérieur (12) de ce filetage se situe à l'état vissé en-dessous du bord supérieur (4) du col (3) du flacon, en ce que le bouchon élastique (7) est disposé à l'intérieur de la partie inférieure (10) de la capsule de telle manière que la face inférieure de sa collerette élastique débordante (8) vienne en contact du talon (12) au-dessus du filetage dans l'état non vissé et que sa section inférieure est dimensionnée de telle façon qu'on puisse l'introduire à force dans le col (3) du flacon, en ce que le support (6) présente sur des faces opposées deux surfaces à garnir avec un milieu de culture solide, est fixé à l'extrémité inférieure du bouchon élastique (7) et s'étend en service sur seulement une partie de la largeur du flacon (2) et en ce que la partie supérieure (13) de la capsule à visser est constituée de telle façon qu'elle comprime fermement à l'état vissé la collerette élastique débordante (8) du bouchon élastique (7) contre le bord supérieur (4) du col (3) du flacon.

2. Flacon pour hémo-culture conforme à la revendication 1 ci-dessus, caractérisé en ce que le col (3) du flacon présente en-dessous de son filetage extérieur (5) un talon (24) formant butée pour la partie inférieure (10) de la capsule vissée.

3. Flacon pour hémo-culture conforme à l'une quelconque des revendications 1 ou 2 ci-dessus, caractérisé en ce que le support (6) présente à son bord supérieur une ou plusieurs, et de préférence deux, rallonges (14) en forme de tiges avec des renflements (15) et en ce que le bouchon élastique (7) présente sur sa face inférieure des trous correspondants (20) dans lesquels ces rallonges (14) sont enfoncées en vue de la fixation.

4. Flacon pour hémo-culture conforme à l'une quelconque des revendications ci-dessus, caractérisé en ce que les surfaces du support (6) à garnir sont lisses.

5. Flacon pour hémo-culture conforme à l'une quelconque des revendications ci-dessus, caractérisé en ce que les surfaces à garnir du support présentent au moins sur leurs deux bords longitudinaux des surfaces de retenue (16) pour les milieux de culture solides, surfaces recouvrant partiellement ces derniers.

6. Flacon pour hémo-culture conforme à l'une quelconque des revendications ci-dessus, caractérisé en ce que le support (6) présente un nez d'égouttage (17).

7. Flacon pour hémo-culture conforme à l'une quelconque des revendications ci-dessus, caractérisé en ce que le milieu de culture solide (25) porté par le support (6) contient de la gomme Gellan.

8. Flacon pour hémo-culture conforme à la revendication 7 ci-dessus, caractérisé en ce que le milieu de culture solide (25) présent sur le support (6) comporte une première couche en McConkey avec une addition de gomme Gellan et de substances à bas poids moléculaire dont la concentration est de 2 à 8 fois celle de la quantité normale, et une couche de couverture en agar-agar normal et/ou en McConkey de composition normale.

**Claims**

1. Blood culture bottle with integrated subculture, containing a bottle which can be filled with liquid nutrient solution and has a bottle neck having an external thread, has a support which can be inserted into the bottle and is to be coated with solid nutrient medium, has an elastic stopper with projecting rim on one side of it and a screw cap, characterized in that the screw cap (9) consists of a lower screw cap part (10) which is open at the top and bottom and has an internal thread (11) and of an upper screw cap part (13) which is open at the bottom, the internal thread (11) has dimensions such that it can be screwed onto the external thread (5) of the bottle neck (3) in such a way that the upper edge (12) of the thread is located, in the screwed-on state, below the upper edge (4) of the bottle neck (3), the elastic stopper (7) is located in the interior of the lower screw cap part (10) in such a way that the undersurface of its projecting elastic rim (8) rests, in the non-screwed-on state, on the shoulder (12) above the thread, and its lower section has dimensions such that it can be introduced, fitting tightly, into the bottle neck (3), the support (6) has on opposite sides two surfaces to be coated with solid nutrient medium, is fixed at the lower end of the elastic stopper (7) and, in the use state, extends only over a part of the length of the bottle (2), and the upper screw cap part (13) is designed such that, in its state after attachment, it pushes the projecting elastic rim (8) of the elastic stopper (7) firmly against the upper edge (4) of the bottle neck (3).

2. Blood culture bottle according to Claim 1, characterized in that the bottle neck (3) has, below the external thread (5), a shoulder (24) as stop for the lower screw cap part (10).

3. Blood culture bottle according to one of Claims 1 or 2, characterized in that support (6) has on its upper rim one or more, preferably two, rod-like attachments (14) with widenings (15), and the elastic stopper (7) has corresponding drilled holes (20) in its undersurface, into which these attachments (14) are inserted for the purpose of fixing.

4. Blood culture bottle according to one of the preceding Claims, characterized in that the surfaces of the support (6) which are to be coated ar smooth.

5. Blood culture bottle according to one of the preceding Claims, characterized in that the surfaces of the support which are to be coated have, at least on the two long sides, holding surfaces (16) for the nutrient medium, which surfaces intersect with the latter.

6. Blood culture bottle according to one of the preceding Claims, characterized in that the support (6) has a projection (17) for drips.

7. Blood culture bottle according to one of the preceding Claims, characterized in that the solid nutrient medium (25) on the support (6) contains gellan gum.

8. Blood culture bottle according to Claim 7, characterized in that the solid nutrient medium (25) has a first layer applied to the support (6), which layer consists of McConkey with an addition of gellan gum and a concentration which is 2–8 times the normal amount of low molecular weight substances, and has a covering layer which consists of normal agar and/or McConkey in the normal composition.

Fig. 1                    1/4

Fig. 2    2/4

## Fig. 3

3/4

## Fig. 4

SCHNITT A-A

# Fig. 5

4/4

SCHNITT A-A

# Fig. 6